(19) **Europäisches Patentamt / European Patent Office / Office européen des brevets**

(11) **EP 3 643 337 B1**

(12) **EUROPÄISCHE PATENTSCHRIFT**

(45) Veröffentlichungstag und Bekanntmachung des Hinweises auf die Patenterteilung:
**23.02.2022  Patentblatt 2022/08**

(21) Anmeldenummer: **19204565.6**

(22) Anmeldetag: **22.10.2019**

(51) Internationale Patentklassifikation (IPC):
**A61L 31/08** *(2006.01)*     **A61L 31/18** *(2006.01)*

(52) Gemeinsame Patentklassifikation (CPC):
**A61L 31/088; A61L 31/18**

(54) **STENT ZUR IMPLANTIERUNG IN EINEN HOHLRAUM EINES MENSCHLICHEN ODER TIERISCHEN KÖRPERS SOWIE VERFAHREN ZUR HERSTELLUNG EINES RÖNTGENOPAKEN SCHICHTAUFBAUS AUF EINEM STENT**

STENT FOR IMPLANTING INTO A CAVITY OF A HUMAN OR ANIMAL BODY AND METHOD FOR PRODUCING AN X-RAY-OPAQUE LAYER SUSPENSION ON A STENT

STENT POUR L'IMPLANTATION DANS UNE CAVITÉ D'UN CORPS HUMAIN OU ANIMAL AINSI QUE PROCÉDÉ DE FABRICATION D'UNE STRUCTURE DE COUCHES OPAQUE AUX RAYONS X SUR UN STENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.10.2018  DE 102018218130**

(43) Veröffentlichungstag der Anmeldung:
**29.04.2020  Patentblatt 2020/18**

(73) Patentinhaber: **Karlsruher Institut für Technologie**
**76131 Karlsruhe (DE)**

(72) Erfinder:
• **Seifried, Fabian**
  **75249 Kieselbronn (DE)**
• **Stüber, Michael**
  **76829 Landau (DE)**
• **Leiste, Harald**
  **76356 Weingarten (DE)**
• **Ulrich, Sven**
  **76297 Stutensee (DE)**
• **Mumbauer, Steffen**
  **76351 Linkenheim-Hochstetten (DE)**
• **Zils, Stefan**
  **76344 Eggenstein-Leopoldshafen (DE)**

(74) Vertreter: **Altmann Stößel Dick Patentanwälte PartG mbB**
**Theodor-Heuss-Anlage 2**
**68165 Mannheim (DE)**

(56) Entgegenhaltungen:
WO-A1-2012/125676     WO-A1-2015/168016
WO-A2-2004/030578     DE-A1- 19 846 013
US-A1- 2002 138 130

EP 3 643 337 B1

**Beschreibung**

Gebiet der Erfindung

Die vorliegende Erfindung betrifft einen Stent zur Implantierung in einen Hohlraum eines menschlichen oder tierischen Körpers sowie ein Verfahren zur Herstellung eines röntgenopaken Schichtaufbaus auf einem Stent. Die Erfindung ist auf dem Gebiet der Medizintechnik angesiedelt.

Stand der Technik

Jährlich werden etwa 220000 Koronarstents und etwas 330000 vaskuläre Stents allein in Deutschland implantiert. Stents werden üblicherweise aus reinem oder beschichtetem Edelstahl, Tantal, einer Kobalt-Chrom-Legierung, einem Biopolymer oder einer Nickel-Ti tan-Legierung (Nitinol) mit Formgedächtniseigenschaften hergestellt. Stents sind grundsätzlich mit unterschiedlichen Stegstrukturen und Designs verfügbar, die einem jeweiligen Einsatzort angepasst sein können. Einfache Stent-Strukturen werden üblicherweise aus Draht geflochten oder geformt. Komplexe Gitterstrukturen werden grundsätzlich mit einem Laser aus einem festen Röhrchen geschnitten, aufgedehnt, partikelbestrahlt und elektropoliert.

Röntgenstrahlung kann Materie grundsätzlich durchdringen und wird dabei je nach Werkstoffart unterschiedlich stark geschwächt. Die Schwächung der Röntgenstrahlen ist ein wichtiger Faktor bei einer radiologischen Bilderzeugung. Eine Intensität I des Röntgenstrahls nimmt mit einer im Werkstoff zurückgelegten Weglänge d nach dem Lambert-Beerschen Gesetz exponentiell ab:

$$I = I_0 e^{-kd}$$

Der Koeffizient k ist ein materialabhängiger Koeffizient. Es gilt:

$$k \sim Z^4 \lambda^3$$

Dabei ist Z eine Ordnungszahl des Werkstoffs und λ eine Wellenlänge des Röntgenstrahls. Aufgrund einer geringen Größe sowie einer geringen Materialdicke können Stents jedoch grundsätzlich in einem Röntgenbild in der medizinischen Bildgebung nur schwer lokalisiert werden. Dies gilt insbesondere bei kleinen Stents mit geringen geometrischen Abmessungen. Eine Implantation solcher Stents ist daher oft schwierig. Eine Position des Stents in einem Körper eines Patienten kann nach einem Freisetzen des Stents nicht mehr korrigiert werden. Daher ist es wichtig, vor der Freisetzung des Stents eine exakte Position des Stents zu sehen und zu beurteilen, insbesondere während einer Operation. Auch eine medizinische Überwachung nach der Implantation um beispielsweise eine Dislokalisation, eine Beschädigung, oder weitere medizinische Effekte zu überprüfen, ist aufgrund der geringen Größe des Stents und der daraus resultierenden geringen Sichtbarkeit in radiologischen Aufnahmen schwierig, oft sogar unmöglich. Für ein Einsetzen eines Stents in den Körper des Patienten ist daher eine Sichtbarmachung im Röntgenbild erforderlich.

Derzeit werden bei Stents zur Erhöhung der Röntgensichtbarkeit Markerpunkte angebracht, beispielsweise durch punktuelles Anschweißen von Kontrastpunkten. Üblicherweise sind die Markerpunkte aus Metallen mit einer höheren Ordnungszahl wie beispielsweise Tantal oder Gold hergestellt. Eine Bildgebung basiert darauf, dass diese Markerpunkte die Röntgenstrahlen stärker absorbieren als das übrige Stentmaterial und dadurch besser sichtbar sind. In C. Bechtold, R. Lima de Miranda, C. Chluba, C. Zamponi, E. Quandt, Method for Fabricating Miniaturized NiTi Self-Expandable Thin Film Devices with Increased Radiopacity, Shap. Mem. Superelasticity 2 (2016) 391-398, wird eine punktuelle, inbesondere lokale, Beschichtung eines Stens mit Tantal in Form von Punkten beschrieben.

Ein weiterer Ansatz, die Röntgensichtbarkeit von Stents zu verbessern besteht darin, die metallischen Stentwerkstoffe wie beispielsweise Nickel-Titan-Legierungen selbst zu modifizieren. Beispielsweise werden Nickel-Titan-Wolfram Legierungen eingesetzt. Dies ist in A. C. Kneissl, K. Mehrabi, M. Bruncko, B. J. McKay, and D. Uhlenhaut, Characterization and properties of NiTi(W) and CuAlNi shape memory alloys, International Journal of Materials Research: Vol 100, No. 8 (2009), 1038-1045, beschrieben. Das Beschichten von Stents zur Verbesserung der Röntgensichtbarkeit kann auch mit röntgenopaken Metallpartikeln erfolgen, die in einem polymeren Bindermaterial eingebettet sind.

In C. Park, S. Kim, H.-E. Kim, T.-S. Jang, Mechanically stable tantalum coating on a nano-roughened NiTi stent for enhanced radiopacity and biocompatibility, Surface and Coatings Technology 305 (2016), 139-145, wird eine Beschichtung eines Stents mit Tantal-Schichten mit einer Dicke von 5 μm bis 10 μm beschrieben. Es wurde festgestellt, dass mindestens eine Schichtdicke von 10 μm notwendig ist, um eine signifikante Verbesserung der Röntgensichtbarkeit des beschichteten Stents zu erreichen.

In C. Heßing, J. Frenzel, M. Pohl, S. Shabalovskaya, Effect of martensitic transformation on the performance of coated NiTi surfaces, Materials Science and Engineering: A 486 (2008) 461-469, werden Titan-Schichten mit einer Schichtdicke von 1,1 $\mu$m zur Verbesserung des Korrosionsverhalten der NiTi-Stents vorgeschlagen: Jedoch tolerieren diese Schichten keine Totaldehnung bis zu 8 %.

In EP 0 709 068 A2 wird ein Stent mit einer signifikanten Menge an Masse mindestens an einem Ende davon beschrieben. In einer Ausführungsform ist der Stent mit einem Material wie Gold oder Tantal beschichtet, das ein hohes Atomgewicht aufweist. Die Plattierung kann den gesamten Stent oder nur einen Teil davon abdecken. In einer zweiten Ausführungsform umfassen die Enden des Stents Vorsprünge, die genügend Metall enthalten, um sie für Röntgenstrahlen sichtbar zu machen.

DE 699 133 42 T2 beschreibt einen intravaskulären Metallstent mit einer röhrenförmigen Wand und einer biokompatiblen Beschichtung auf mindestens einem Hauptteil der Wandoberfläche, wobei die Beschichtung eine Dicke von weniger als 4 $\mu$m aufweist und ein diamantartiges amorphes Material, vorzugsweise DLN, enthält.

DE 10 2007 030 751 B4 beschreibt einen Stent. Der Stent hat eine röhrenförmige Gitterstruktur mit einer Stange, die in einem Ausmaß angeordnet ist. Ein verformbarer, röntgennaher Stentkern bildet die Gitterstruktur. Der Stentkern weist eine Teilabdeckung aus einem biokompatiblen Material auf. Die Wanddicke der Abdeckung ist größer als die Breite des Stentkerns. Die Abdeckung besteht aus formstabilem Material, beispielsweise aus Edelstahl, einer Kobalt-Chrom-Legierung oder einer Magnesiumlegierung. Ein galvanisches Formverfahren wird implementiert, um die Belichtung und Bildung einer photoaktiven Substanz zu ermöglichen, so dass die Form des Stentkerns bestimmt wird. Ein unabhängiger Anspruch ist auch für ein Verfahren zum Herstellen eines Stents eingeschlossen.

In EP 0 824 900 A2 werden beschichtete Stents und ein Verfahren zu ihrer Herstellung beschrieben. Ein Stent, der im Wesentlichen strahlendurchlässig ist, ist zumindest teilweise mit einer strahlenundurchlässigen Schicht überzogen, die den Stent unter Röntgenstrahlung oder mittels Fluoroskopie sichtbar macht. Eine Schutzschicht ist auf den Stent und die strahlenundurchlässige Schicht aufgetragen, um sowohl vor Kratzern, Abblättern und galvanischer Korrosion zu schützen und um sowohl die Blut- als auch die Biokompatibilität zu verbessern.

In KR 101 791 337 B1 wird ein Verfahren zur Herstellung eines auf Nitinol basierenden Stents beschrieben, umfassend eine strahlenundurchlässige Schwermetallbeschichtung, die durch ein selektives Plasmaätzverfahren gebildet wird.

In US 6 638 301 B1 werden eine Endoprothese und ein Stent mit einer Schicht, die die Biokompatibilität der Endoprothese verbessern kann, und ein Verfahren zur Herstellung der Endoprothese offenbart.

US2002/138130 A1 beschreibt intravaskuläre Vorrichtungen mit einer darauf befindlichen strahlenundurchlässigen Schicht zur Sichtbarmachung. Die Vorrichtungen umfassen ferner eine Deckschicht auf der strahlenundurchlässigen Schicht, um zu verhindern, dass das strahlenundurchlässige Material dem umgebenden Gewebe ausgesetzt wird. Ein Verfahren zum Beschichten der Vorrichtung wird ebenfalls beschrieben. Das Verfahren umfasst die Verwendung eines Magnetrons mit unausgeglichenem Magnetfeld, um aus einer Quelle Metallatome zum Beschichten zu erzeugen und Ionen zu bombardieren, um abgeschiedene Metallatome auf die Oberfläche der Vorrichtung zu komprimieren.

WO 2015/168016 A1 beschreibt eine implantierbare medizinische Vorrichtung, welche ein Strukturelement mit einer Kernschicht oder einem Bereich aus strahlendurchlässigem Material; eine Schicht aus strahlenundurchlässigem Material, die über der Kernschicht oder dem Bereich aus strahlendurchlässigem Material liegt; und eine äußere Schicht aus strahlendurchlässigem Material, die über der Schicht aus strahlenundurchlässigem Material liegt, umfasst. Die äußere Schicht aus strahlendurchlässigem Material weist eine gleiche oder größere Härte wie die Schicht aus strahlenundurchlässigem Material auf.

Trotz der Vorteile der aus dem Stand der Technik bekannten Vorrichtungen und Verfahren beinhalten diese noch Verbesserungspotenzial.

Bei einem Einsatz von Markerpunkten sind in den meisten Fällen jedoch nur die angebrachten Marker auf dem Röntgenbild zu erkennen. Das Anschweißen der Markerpunkte erfolgt derzeit überwiegend manuell. Der Zeitbedarf dieser höchst anspruchsvollen und konzentrationsintensiven Handarbeit liegt derzeit bei ca. 20 min pro Stent. Ein automatisiertes Anschweißen ist aufgrund der geringen Größe der Markerpunkte (d < 0,1 mm) technisch nur sehr aufwändig möglich. Die manuelle Durchführung dieser Arbeit ist eintönig, erfordert jedoch, wie oben bereits ausgeführt, höchste Aufmerksamkeit und ist dadurch sehr anstrengend und letztlich - für den Patienten - auch stark risikobehaftet. Bei der Verwendung von angeschweißten oder aufgepressten Markerpunkten kann es an den Kontaktstellen zu Korrosion oder langfristig auch zu einer Ablösung der Marker kommen. Dies kann zu Lungenembolien oder sogar zu Schlaganfällen führen. Des Weiteren kann die Sichtbarkeit von Stents mit Markerpunkten im Röntgenbild nicht ausreichend sein, weil die Lage des Stents in einem Gefäß im Körper des Patienten nicht eindeutig zu erkennen ist. Darüber hinaus können kleine Stents nicht auf Bruch- oder sonstige Schadstellen kontrolliert werden, da nur die Markerpunkte sichtbar sind. Fragen nach einer korrekten Lage des Stents, nach Beschädigungen oder gar nach einer Erfüllung der medizinischen Aufgabe (z. B. Einknicken und damit Versagen der Funktion des Stents) können bei kleinen Stents mit Markerpunkten grundsätzlich nicht beantwortet werden.

Eine röntgenopake Beschichtung bietet die Möglichkeit, vorhandene Stentdesigns weiter zu verwenden. Bei der Veränderung des Stentwerkstoffs, beispielsweise der Legierungsart oder -zusammensetzung, müssen die Stentdesigns neu erarbeitet und eventuell auch einem erneuten Zulassungsverfahren unterzogen werden. Dieses Verfahren ist mit hohen Kosten für den Stenthersteller verbunden. Des Weiteren werden beispielsweise Kobalt-Chrom-Legierungen mit einem Medikament beschichtet und sind daher nicht vergleichbar. Befindet sich der röntgenopake·Werkstoff in einem polymeren Bindermaterial, muss eine homogene Partikelverteilung nach dem Beschichtungsvorgang gewährleistet sein. Existiert keine homogene Partikelverteilung ist der Stent wie bei der Markermethode nur punktuell sichtbar.

Einfachschichten, insbesondere Einlagenschichten, wie zum Beispiel Tantal-Schichten, können nach einmaliger mechanischer Belastung im Bereich von 5 bis 10% Totaldehnung eine Rissbildung an der Schichtoberfläche zeigen. Somit ist die Schichtintegrität nicht mehr gewährleistet und das Versagensrisiko bei dynamischer Belastung ist erhöht. Des Weiteren führen die Risse zum Verlust der verbesserten Biokompatibilität und des verbesserten Korrosionsschutzes im Vergleich zum nicht beschichteten Stent, weil der Stent direkt mit dem menschlichen Organismus in Kontakt ist. Titan-Schichten können aufgrund der niedrigen Massendichte nicht die Röntgensichtbarkeit des Stents verbessern und versagen bereits bei einer Totaldehnung von 1 %. Einfachschichten zur Erhöhung der Röntgensichtbarkeit sind grundsätzlich nicht in der Lage, die hohen mechanischen Belastungen, die ein Stent während seiner Lebensdauer durchläuft, zu tolerieren. Eine vollständige Beschichtung des Stents mit Gold ist aus wirtschaftlicher Sicht nicht möglich, weil die Kosten für die Stentproduktion den Verkaufspreis für den Stent übersteigen würden. Damit ist dieser Schichtwerkstoff für ein kommerziell agierendes Unternehmen nicht relevant. Ein teilweises Beschichten von Stents führt wie bei der Markermethode, nur zu einer teilweisen Sichtbarkeit des Stents auf röntgenographischen Aufnahmen. Somit ergibt sich kein Vorteil gegenüber der Markermethode und es können auch keine Kosten eingespart werden.

Aufgabe der Erfindung

Ausgehend hiervon besteht die Aufgabe der vorliegenden Erfindung darin, einen Stent zur Implantierung in einen Hohlraum eines menschlichen oder tierischen Körpers sowie ein Verfahren zur Herstellung eines röntgenopaken Schichtaufbaus auf einem Stent bereitzustellen, welche die aufgeführten Nachteile und Einschränkungen des Standes der Technik zumindest teilweise überwinden. Insbesondere soll der Stent sowie das Verfahren zur Herstellung eines röntgenopaken Schichtaufbaus auf einem Stent eine Sichtbarkeit des Stents im Rahmen einer medizinischen, insbesondere röntgenographischen, Bildgebung erhöhen.

Offenbarung der Erfindung

Diese Aufgabe wird durch einen Stent zur Implantierung in einen Hohlraum eines menschlichen oder tierischen Körpers gemäß Anspruch 1 sowie durch ein Verfahren zur Herstellung eines röntgenopaken Schichtaufbaus auf einem Stent gemäß Anspruch 9 gelöst. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elements. Bei einer nachfolgenden erneuten Erwähnung des Merkmals oder Elements wird der entsprechende Begriff "mindestens ein" oder "ein oder mehrere" in der Regel nicht mehr verwendet, ohne dass hierdurch die Möglichkeit eingeschränkt wird, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten unangetastet bleiben, die hierdurch eingeleiteten Merkmale mit anderen Merkmalen zu kombinieren, seien es optionale oder nicht-optionale Merkmale.

Die Bezeichnungen "erste/r" und "zweite/r" sind als reine Beschreibungen anzusehen, ohne eine Reihenfolge oder Rangfolge anzugeben und beispielsweise ohne die Möglichkeit auszuschließen, dass mehrere Arten von ersten Elementen beziehungsweise zweiten Elementen oder jeweils genau eine Art vorgesehen sein kann. Weiterhin können zusätzliche Elemente, beispielsweise eine oder mehrere dritte Elemente vorhanden sein.

Die Erfindung wird durch die Ansprüche definiert.

In einem ersten Aspekt der vorliegenden Erfindung wird ein Stent zur Implantierung in einen Hohlraum eines menschlichen oder tierischen Körpers vorgeschlagen. Der Stent umfasst mindestens einen röhrenförmigen Hohlkörper. Der röhrenförmige Hohlkörper weist mindestens eine Oberfläche auf, welche vollständig mit einem röntgenopaken Schichtaufbau beschichtet ist. Der röntgenopake Schichtaufbau weist eine Mehrzahl an jeweils übereinanderliegenden Schichten auf, wobei der röntgenopake Schichtaufbau mindestens zehn Schichten aufweist. Die Schichten sind jeweils aus mindestens einem Metall hergestellt, welches eine Ordnungszahl von 22 bis 83 aufweist, wobei das Metall ausgewählt ist aus der Gruppe bestehend aus Wolfram, Niob, Tantal und Titan.

Weiterhin weisen die Schichten jeweils eine Schichtdicke im Bereich von 1 nm bis 400 nm, vorzugsweise von 2 nm bis 200 nm, auf. Insbesondere kann jede Schicht des röntgenopaken Schichtaufbaus jeweils die Schichtdicke im Bereich von 1 nm bis 400 nm, vorzugsweise von 2 nm bis 200 nm, aufweisen.

Der Begriff "Stent" bezeichnet grundsätzlich ein beliebiges medizinisches Implantat zum Offenhalten von Gefäßen oder Hohlorganen eines menschlichen oder tierischen Körpers. Der Stent kann daher auch als Gefäßstütze bezeichnet werden. Der Stent kann eingerichtet sein, um in Blutgefäßen, speziell Herzkranzgefäßen, eingebracht zu werden, insbesondere um nach einer Aufdehnung des Stents einen erneuten Verschluss zu verhindern. Eine solche Behandlung wird als Stentangioplastie bezeichnet. Weiterhin kann der Stent eingerichtet sein für einen Einsatz in einer Krebsbehandlung. Dabei kann der Stent eingerichtet sein, durch bösartige Tumore verursachte Verengungen von Atemwegen, insbesondere der Luftröhre, der Gallenwege oder der Speiseröhre nach einer Aufdehnung offenzuhalten. Der Begriff "Hohlraum des menschlichen oder tierischen Körpers" bezeichnet daher insbesondere ein Gefäß oder ein Hohlorgan des menschlichen oder tierischen Körpers.

Wie bereits oben ausgeführt, umfasst der Stent mindestens einen röhrenförmigen Hohlkörper. Der Begriff "Hohlkörper" bezeichnet grundsätzlich ein beliebiges Element, welches einen Hohlraum aufweist. Unter einem "Hohlraum" wird im Rahmen der vorliegenden Erfindung grundsätzlich ein beliebiger in mindestens einer Ebene vollständig umschlossener Bereich verstanden. Der vollständig umschlossene Bereich kann jedoch ein oder mehrere lokale Öffnungen aufweisen. Der Begriff "röhrenförmiger Hohlkörper" bezeichnet grundsätzlich, dass der Hohlkörper eine längliche Form aufweisen kann. Eine Länge des röhrenförmigen Hohlkörpers kann daher größer sein als ein Durchmesser des röhrenförmigen Hohlkörpers. Insbesondere kann eine Ausdehnung des röhrenförmigen Hohlkörpers in lateraler Dimension eine Dicke des röhrenförmigen Hohlkörpers überschreiten, beispielsweise um einen Faktor von mindestens 2, vorzugsweise um einen Faktor von mindestens 5. Der röhrenförmige Hohlkörper kann eine zylindrische Grundform aufweisen. Der röhrenförmige Hohlkörper kann daher auch als Hohlzylinder bezeichnet werden.

Der Stent kann aus mindestens einem Material hergestellt sein, ausgewählt aus der Gruppe bestehend aus: einem Edelstahl; einer Formgedächtnislegierung. Der Edelstahl kann insbesondere ein austenitischer, rostfreier Stahl sein und die Werkstoffnummer 1.4404 (X2CrNiMo17-12-2) sowie die AISI Werkstoffbezeichnung 316L aufweisen. Die Formgedächtnislegierung kann ausgewählt sein aus der Gruppe bestehend aus: einer Nickel-Titan-Legierung; einer Nickel-Titan-X-Legierung, wobei X insbesondere ein Metall, vorzugsweise Wolfram ist; einer Kobalt-Chrom-Legierung; einer beta-Titan-Legierung.

Insbesondere kann es sich bei dem Stent um eine Spiraldrahtprothese handeln. Der Begriff "Spiraldrahtprothese" bezeichnet insbesondere, dass das Material des Stents in einer Drahtform bereitgestellt wird. Einzelne Drähte des Stents können sich quer, insbesondere senkrecht, zu einer Längserstreckungsachse des Stents erstrecken und so den Hohlkörper ausbilden. Die Drähte können daher eine Mantelfläche des Hohlkörpers bilden. Die Drähte können derart angeordnet sein, dass sich zwischen den Drähten Hohlräume ausbilden. Die Mantelfläche kann daher als offene Mantelfläche ausgebildet sein.

Der Stent kann insbesondere eine innere Oberfläche und eine äußere Oberfläche aufweisen. Die innere Oberfläche kann einem Innenraum des röhrenförmigen Hohlkörpers zugewandt sein und die äußere Oberfläche kann einer äußeren Umgebung des röhrenförmigen Hohlkörpers zugewandt sein. Der Begriff "Oberfläche" im Sinne der vorliegenden Erfindung kann daher sowohl die innere Oberfläche als auch die äußere Oberfläche umfassen. Weiterhin kann der Stent, wie oben beschrieben, aus Drähten hergestellt sein. In diesem Fall kann der Begriff "Oberfläche" im Sinne der vorliegenden Erfindung eine umlaufende Oberfläche der Drähte umfassen. Insbesondere kann der Begriff "Oberfläche" eine Mantelfläche der Drähte umfassen.

Wie oben bereits ausgeführt, ist die Oberfläche vollständig mit dem röntgenopaken Schichtaufbau beschichtet. Dem Fachmann ist jedoch bekannt, dass es, insbesondere produktionsbedingt, zu leichten Abweichungen von einer vollständigen Bedeckung der Oberfläche mit dem röntgenopaken Schichtaufbau kommen kann. Der Begriff "vollständig" beinhaltet daher leichte Abweichungen von einer vollständigen Bedeckung der Oberfläche mit dem röntgenopaken Schichtaufbau. Insbesondere kann der Begriff "vollständig" auch eine Bedeckung von mindestens 80%, vorzugsweise von mindestens 90 % und besonders bevorzugt von mindestens 95 % der Oberfläche mit dem röntgenopaken Schichtaufbau beinhalten.

Der Begriff "Schicht" bezeichnet grundsätzlich ein beliebiges Element mit einer länglichen Form und einer Dicke, wobei eine Ausdehnung des Elements in lateraler Dimension eine Dicke des Elements überschreitet, beispielsweise um einen Faktor von 10 bis 1000, vorzugsweise um einen Faktor von 200 bis 500. Die Schicht kann auf einem Substrat aufgebracht sein oder auf einer weiteren Schicht. Die Schicht kann eine durchgängige Schicht sein. Alternativ kann die Schicht jedoch an ein oder mehreren Stellen unterbrochen sein, beispielsweise durch Vertiefungen oder Risse. Der Begriff "Substrat" bezeichnet grundsätzlich ein beliebiges Element, welches eingerichtet ist, ein oder mehrere weitere Elemente zu tragen und welches dementsprechend eine mechanische Stabilität aufweist. Die Oberfläche des röhrenförmigen Hohlkörpers kann das Substrat für den röntgenopaken Schichtaufbau ausbilden.

Unter einem "Schichtaufbau" ist im Rahmen der vorliegenden Erfindung grundsätzlich eine Abfolge von mindestens zwei Schichten, welche direkt oder unter Zwischenschaltung einer oder mehrerer Zwischenschichten aufeinander aufgebracht sind, zu verstehen. Der Schichtaufbau kann mehrere Schichten desselben Materials aufweisen. Weiterhin kann der Schichtaufbau Schichten verschiedener Materialien aufweisen. Auch andere Ausführungsformen sind grundsätzlich denkbar. Der Schichtaufbau weist mindestens zehn Schichten auf. Der Schichtaufbau kann daher auch als Viellagenschicht oder als Viellagenschichtaufbau bezeichnet werden. Die Schichten können durch Grenzflächen voneinander abgegrenzt sein.

Der Begriff "übereinanderliegend" bezeichnet grundsätzlich eine Lage einer Oberfläche zu einer anderen Oberfläche, wobei die beiden Oberflächen gegenüberliegend angeordnet sind. Hierbei können Abweichungen von einem Aneinandergrenzen der beiden Oberflächen auftreten. Die Abweichungen können beispielsweise maximal 20 %, vorzugsweise maximal 10 % und besonders bevorzugt maximal 5 % betragen. Eine Schicht des Schichtaufbaus kann insbesondere mindestens eine Längsseite und mindestens eine Stirnseite aufweisen, wobei die Längsseite länger ist als die Stirnseite. Eine erste Oberfläche einer ersten Längsseite einer ersten Schicht des Schichtaufbaus kann einer zweiten Oberfläche einer zweiten Längsseite einer zweiten Schicht des Schichtaufbaus gegenüberliegen. Insbesondere können die erste Oberfläche und die zweite Oberfläche in einem direkten Kontakt zueinander stehen. Insbesondere kann die zweite Schicht auf der ersten Schicht aufliegen, wobei sich die erste Oberfläche und die zweite Oberfläche zumindest teilweise berühren. In einer derartigen Anordnung kann die zweite Schicht beispielsweise kleinere Dimensionen, insbesondere eine kleinere Länge und/oder Breite, aufweisen als die erste Schicht oder umgekehrt. Dabei können Teile der zweiten Oberfläche von der ersten Schicht unbedeckt sein oder umgekehrt. Weiterhin können die erste Schicht und die zweite Schicht zueinander versetzt angeordnet sein, d.h. ein Teil der zweiten Schicht kann über einen Rand der ersten Schicht herausragen oder umgekehrt.

Der Begriff "röntgenopak" bezeichnet grundsätzlich eine Eigenschaft einer Strahlenundurchlässigkeit oder einer Strahlenschwächung von Materialen für Röntgenstrahlen. Das Metall kann daher mindestens einen linearen Schwächungskoeffizienten im Bereich von 5,46 $cm^{-1}$ bis 149,08 $cm^{-1}$ bei 50 eV aufweisen. Wie bereits oben ausgeführt, weist das Metall eine Ordnungszahl von 22 bis 83 auf. Vorzugsweise ist das Metall biokompatibel. Das Metall ist ausgewählt aus der Gruppe bestehend aus: Wolfram, Niob, Tantal, Titan. Diese Metalle können in einer kubisch raumzentrierten Kristallstruktur vorliegen. Eine oder mehrere der Schichten, vorzugsweise alle der Schichten, können jeweils insbesondere eine einphasige kubisch raumzentrierte Kristallstruktur aufweisen.

In einer oder mehreren der Schichten kann das Metall als Reinstoff vorliegen. Alternativ und/oder zusätzlich können eine oder mehrere der Schichten jeweils mindestens eine Legierung aufweisen. Die Legierung kann ausgewählt sein aus der Gruppe bestehend aus: einer einphasigen binären $Ta_{1-x}Nb_x$-Legierung mit $0<x<l$, vorzugsweise mit $0,28<x<1$ und besonders bevorzugt mit $0,28<x<0,65$; einer einphasigen binären $Ti_{1-x}Ta_x$-Legierung mit $0<x<l$, vorzugsweise mit $0<x<0,56$; einer ternären $Ta_{1-x-y}Ti_xNb_y$-Legierung mit $0<x<1$ und mit $0<y<l$, vorzugsweise mit $0<x<0,7$ und mit $0<y<0,7$, besonders bevorzugt mit $0<x<0,56$ und mit $0<y<0,6$.

Binäre Phasendiagramme von Legierungen aus Wolfram, Niob, Tantal und/oder Titan können jeweils vollständige Löslichkeiten ineinander zeigen. Binäre Legierungen, beispielsweise Legierungen umfassend Tantal und Niob, lassen sich daher in einer beliebigen Konzentration mit einer entsprechenden Kristallstruktur darstellen, vorzugsweise jedoch in einer kubisch raumzentrierten Kristallstruktur. Bei Titanlegierungen kann eine Legierung mit einem Metall, welches eine kubisch raumzentrierte Kristallstruktur aufweist, zu einer Stabilisierung einer duktilen beta-Titanphase führen. Dies resultiert in ein pseudo-elastisches Werkstoffverhalten für beta-Titanlegierungen. Ternäre Legierungssysteme, beispielsweise umfassend Titan, Tantal und Niob, können ebenfalls einphasige Mischkristalle sowie eine kubisch raumzentrierte Kristallstruktur ausbilden.

Insbesondere kann der röntgenopake Schichtaufbau einen Gradienten aufweisen, wobei sich die Schichten des opaken Schichtaufbaus in Konzentrationen von Komponenten der Legierungen unterscheiden. Eine erste Konzentration einer ersten Komponente kann mit einem zunehmenden Abstand einer der Schichten von der Oberfläche des röhrenförmigen Hohlkörpers abnehmen und eine zweite Konzentration einer zweiten Komponente kann mit einem zunehmenden Abstand einer der Schichten von der Oberfläche des röhrenförmigen Hohlkörpers zunehmen. Beispielsweise kann eine an die Oberfläche des Stents angrenzende Schicht eine Zusammensetzung $Ta_{0,9}Nb_{0,1}$ aufweisen und eine an eine äußere Umgebung des Stents angrenzende Schicht eine Zusammensetzung $Ta_{0,1}Nb_{0,9}$. Eine Verwendung von Tantal und Niob kann vorteilhaft sein, da Tantal und Niob grundsätzlich vollständig ineinander löslich sind. Eine vollständige Löslichkeit kann auch bei folgenden Legierungssystemen gegeben sein: NbTi, NbW, TaW. Diese können ebenfalls für den röntgenopaken Schichtaufbau eingesetzt werden.

Der Begriff "Gradient" bezeichnet grundsätzlich ein Gefälle oder einen Anstieg einer beliebigen Größe oder eines beliebigen Parameters zwischen zwei Orten. Insbesondere kann es sich um einen Konzentrationsgradienten zwischen den beiden Orten handeln, wobei sich eine erste Konzentration eines Stoffes an einem ersten Ort von einer zweiten Konzentration an einem zweiten Ort unterscheidet. Insbesondere kann anstelle einer scharf definierten Grenzfläche zwischen dem ersten Ort und dem zweiten Ort ein kontinuierlicher Übergang vorliegen. Der Gradient kann insbesondere von der einer äußeren Umgebung zugewandten Oberfläche eines Körpers in ein Inneres des Körpers abwärts oder aufwärts gerichtet sein. Folglich kann eine erste Konzentration eines ersten Stoffes an oder in Nähe der äußeren Oberfläche größer sein als eine zweite Konzentration eines zweiten Stoffes in dem Inneren des Körpers.

Wie bereits oben ausgeführt, weisen die Schichten jeweils eine Schichtdicke von 1 nm bis 400 nm auf. Vorzugsweise kann die Schichtdicke im Bereich von 2 nm bis 50 nm, besonders bevorzugt im Bereich von 2 nm bis 8 nm liegen. Insbesondere können die Schichten jeweils eine Schichtdicke von 5 nm bis 250 nm, vorzugsweise von 8 nm bis 100 nm, vorzugsweise von 10 nm bis 50 nm, besonders bevorzugt von 20 nm bis 40 nm, aufweisen. Insbesondere können die Schichten jeweils eine Schichtdicke von 2 nm bis 100 nm, vorzugsweise von 2 nm bis 50 nm, vorzugsweise von 2 nm bis 10 nm, besonders bevorzugt von 1 nm bis 10 nm, aufweisen. Weiterhin können die Schichten jeweils eine Schichtdicke von 1 nm bis 5 nm, vorzugsweise von 2 nm bis 4 nm, aufweisen. Weiterhin können die Schichten jeweils eine Schichtdicke von 4 nm bis 10 nm, vorzugsweise von 4 nm bis 8 nm, und besonders bevorzugt von 4 nm bis 6 nm aufweisen. Eine Schichtdicke der Schichten von jeweils 5 nm bis 10 nm ist ebenfalls denkbar. Die Schichtdicken der einzelnen Schichten können jeweils gleich groß oder auch voneinander verschieden sein. Insbesondere kann jede Schicht des röntgenopaken Schichtaufbaus jeweils die Schichtdicke in einem der oben genannten Intervalle aufweisen. Der röntgenopake Schichtaufbau kann eine Schichtaufbaudicke von 500 nm bis 100 $\mu$m aufweisen, vorzugsweise von 2 $\mu$m bis 50 $\mu$m vorzugsweise von 1 $\mu$m bis 20 $\mu$m, besonders bevorzugt 10 $\mu$m. Auch andere Dimensionen sind jedoch grundsätzlich denkbar.

Insbesondere kann der röntgenopake Schichtaufbau mindestens zwei erste Schichten mit einem ersten Material und mindestens eine zweite Schicht mit einem zweiten Material aufweisen. Die zweite Schicht kann zwischen den ersten Schichten angeordnet sein. Weiterhin kann der röntgenopake Schichtaufbau eine Vielzahl der ersten Schichten und eine Vielzahl der zweiten Schichten aufweisen, welche jeweils abwechselnd übereinander angeordnet sind, das heißt zwischen zwei der ersten Schichten kann eine der zweiten Schichten angeordnet sein und zwischen zwei der zweiten Schichten kann eine der ersten Schichten angeordnet sein. Das erste Material und das zweite Material können voneinander verschieden sein. Insbesondere können die ersten Schichten Tantal aufweisen und die zweite Schicht kann Titan oder Niob aufweisen. Auch andere Materialien sind jedoch grundsätzlich denkbar, wobei das Metall ausgewählt ist aus der Gruppe bestehend aus: Wolfram, Niob, Tantal, Titan. Die ersten Schichten und/oder die zweiten Schichten können jeweils eine Schichtdicke von 2 nm bis 8 nm aufweisen. Somit kann eine Übergitterstruktur, d.h. ein Festkörper, der eine Abfolge von dünnen Schichten aufweist, die sich periodisch wiederholen, erzeugt werden, die das elastische und plastische Werkstoffverhalten des Schichtaufbaus positiv beeinflusst. Dies kann beispielsweise eine Erhöhung einer Streckgrenze, eine Steigerung einer Bruchdehnung und/oder eine Erniedrigung eines Elastizitätsmoduls umfassen.

Weiterhin kann eine erste Schicht des Schichtaufbaus eine erste Schichtdicke $d_1$ aufweisen und eine zweite Schicht des Schichtaufbaus kann eine zweite Schichtdicke $d_2$ aufweisen, wobei ein Verhältnis $d_1:d_2$ mittels eines Beschichtungsprozesses einstellbar ist. Somit können die mechanischen Eigenschaften und/oder die Röntgensichtbarkeit des Stents individuell angepasst werden. Beispielsweise kann das Verhältnis 10:1 betragen. Hierbei kann die erste Schichtdicke $d_1$ 100 nm betragen und die zweite Schichtdicke $d_2$ kann 10 nm betragen. Das Verhältnis $d_1:d_2$ kann jedoch auch 1:1 sein, d.h. die erste Schichtdicke und die zweite Schichtdicke $d_2$ können gleich groß sein.

In einem weiteren Aspekt der vorliegenden Erfindung wird ein Verfahren zur Herstellung eines röntgenopaken Schichtaufbaus auf einem Stent zur Implantierung in einen Hohlraum eines menschlichen oder tierischen Körpers vorgeschlagen. Das Verfahren kann die Verfahrensschritte, welche im Folgenden beschrieben werden, umfassen. Die Verfahrensschritte können insbesondere in der vorgegebenen Reihenfolge durchgeführt werden. Eine andere Reihenfolge ist jedoch ebenfalls denkbar. Weiterhin können ein oder mehrere Verfahrensschritte gleichzeitig oder zeitlich überlappend durchgeführt werden. Weiterhin können einer, mehrere oder alle der Verfahrensschritte einfach oder auch wiederholt durchgeführt werden. Das Verfahren kann darüber hinaus noch weitere Verfahrensschritte umfassen.

Das Verfahren umfasst die folgenden Schritte:

a) Bereitstellen eines röhrenförmigen Hohlkörpers, wobei der röhrenförmige Hohlkörper mindestens eine Oberfläche aufweist; und

b) Aufbringen eines röntgenopaken Schichtaufbaus auf die Oberfläche, derart, dass die Oberfläche vollständig bedeckt wird, wobei der röntgenopake Schichtaufbau eine Mehrzahl an jeweils übereinanderliegenden Schichten aufweist, wobei der röntgenopake Schichtaufbau mindestens zehn Schichten aufweist, wobei die Schichten jeweils aus mindestens einem Metall hergestellt sind, welches eine Ordnungszahl von 22 bis 83 aufweist, wobei das Metall ausgewählt ist aus der Gruppe bestehend aus: Wolfram, Niob, Tantal, Titan, wobei die Schichten jeweils eine Schichtdicke d im Bereich von 1 nm bis 400 nm aufweisen.

Der Begriff "Bereitstellen" bezeichnet grundsätzlich einen beliebigen Vorgang, bei welchem ein beliebiger Gegenstand, insbesondere ein beliebiges Material, zur Verfügung gestellt wird, sodass der Gegenstand bzw. das Material für eine Verarbeitung verwendet werden kann. Das Bereitstellen kann auch ein Herstellen des Gegenstands bzw. des Materials umfassen.

Der Begriff "Aufbringen" bezeichnet grundsätzlich einen beliebigen Vorgang, bei welchem ein Material auf eine beliebige Oberfläche eines weiteren Elements angebracht wird. Insbesondere kann das Material derart auf die Oberfläche angebracht werden, dass das Material anschließend, gegebenenfalls auch durch einen nachfolgenden Trocknungsschritt, an der Oberfläche haftet. Das Aufbringen kann auch ein Beschichten der Oberfläche mit dem Material sein oder umfassen. Der Begriff "Beschichten" bezeichnet grundsätzlich ein Aufbringen einer Schicht, insbesondere einer festhaftenden Schicht, aus einem formlosen Stoff auf eine beliebige Oberfläche eines Elements oder Werkstücks. Das Beschichten kann ein Aufbringen einer einzelnen Schicht oder auch ein Aufbringen von mehreren Schichten, insbesondere von mehreren übereinanderliegenden Schichten, umfassen.

Die Schichten können jeweils nacheinander mittels physikalischer Gasphasenabscheidung hergestellt werden, vorzugsweise mittels mindestens eines der folgenden Verfahren: Hochleistungskathodenzerstäubung, Hochfrequenz-Magnetron-Hochleistungs-KathodenZerstäubung, bevorzugt bei 13,56 MHz, insbesondere Gleichspannung-Magnetron-Hochleistungskathodenzerstäubung, insbesondere gepulste Gleichspannung-Magnetron-Hochleistungskathodenzerstäubung; Gleichspannung-Lichtbodenverdampfung; Laserablation. Die gepulste Gleichspannung-Magnetron-Hochleistungskathodenzerstäubung kann Hochleistungsimpulsmagnetronsputtern (HIPIMS) umfassen. Weiterhin können die Schichten mittels Mittelfrequenz-Magnetron-Hochleistungskathodenzerstäubung, bevorzugt bei 20-30 kHz, hergestellt werden. Alle diese Verfahren sind einzeln oder in jeder Kombination anwendbar. Auch andere Verfahren sind jedoch grundsätzlich denkbar. Die Schichtabscheidung kann insbesondere mit reinen Ta, Nb, W- und/oder Ti-Targets erfolgen. Darüber hinaus kann die Schichtabscheidung mit TiTa-, TiW- und/oder TaNb-Legierungstargets mit unterschiedlicher Zusammensetzung erfolgen. Die physikalische Gasphasenabscheidung kann in einem Niedertemperaturplasma mit einer Temperatur von vorzugsweise 100 °C bis 200 °C, besonders bevorzugt von 150 °C, erfolgen. Hierdurch kann eine Veränderung einer Kristallstruktur des Materials des röhrenförmigen Hohlkörpers vermieden oder zumindest weitgehend reduziert werden. Insbesondere kann eine Veränderung der Kristallstruktur von NiTi-Legierungen vermieden werden.

Weiterhin kann eine Abscheiderate einer der Schichten des röntgenopaken Schichtaufbaus auf der Oberfläche des röhrenförmigen Hohlkörpers oder auf einer darunterliegenden Schicht des Schichtaufbaus erhöht werden durch einen Einsatz von Mikrowellenstrahlung. Die Mikrowellenstrahlung wird genutzt, um ein Niedertemperatur-Plasma in einer Beschichtungskammer zu beeinflussen. Über grundsätzliche plasmaphysikalische Zusammenhänge können dann physikalische Kenngrößen des Niedertemperatur-Plasmas und von für eine Schichtbildung benötigten Teilchen gezielt beeinflusst werden.

Die physikalische Gasphasenabscheidung kann unter Anwendung einer Substratvorspannung erfolgen. Dadurch können Druckspannungen in den Schichten zur Kompensation von Zugsspannungen gezielt induziert werden. Bei der Substratvorspannung kann ein Gradientenkonzept eingesetzt werden. Dabei kann eine Veränderung der Substratvorspannung in festgelegten Zeitintervallen erfolgen. Die Schichten können jeweils zunächst ohne zusätzlichen Ionenbeschuss aufwachsen. Ein Einfluss eines Substratvorspannung-induzierten Ionenbeschusses kann mit wachsender Schichtdicke zunehmen. In einem mittleren und in einem oberflächennahen Bereich der Schicht kann eine Kristallitgröße der Schicht beispielsweise reduziert werden. Aufgrund einer Kornfeinung kann es zu einem leichteren Abgleiten der Körner kommen und die Schicht kann eine bessere Verformbarkeit unter mechanischer Belastung zeigen.

Nach dem Aufbringen des röntgenopaken Schichtaufbaus, insbesondere nach einem Aufbringen einzelner Schichten, kann eine Wärmebehandlung erfolgen. Dadurch können im Bereich der Grenzflächen zwischen zwei der Schichten Legierungen von benachbarten Metallen gebildet werden. Dies kann grundsätzlich zu einer Verbesserung des elastischen und/oder plastischen Materialverhaltens des Schichtaufbaus beitragen.

Der vorgeschlagene Stent und das vorgeschlagene Verfahren weisen gegenüber bekannten Vorrichtungen und Verfahren zahlreiche Vorteile auf.

Insbesondere kann eine Sichtbarkeit des Stens sowohl während einer Operation am menschlichen oder tierischen Körper als auch nach der Operation verbessert werden.

Um eine gute Sichtbarmachung mittels Röntgenstrahlung zu erreichen, wird der komplette Stent mit metallischen Werkstoffen dauerhaft beschichtet. Eine Sichtbarkeit in der Röntgenstrahlung wird über einen Massenkontrast erreicht, das heißt letzten Endes über ein Volumen des Werkstoffes, der die Röntgensichtbarkeit ermöglicht. Durch einen Einsatz von Metallen mit Ordnungszahlen von 22 bis 83, wobei das Metall ausgewählt ist aus der Gruppe bestehend aus Wolfram, Niob, Tantal und Titan, kann grundsätzlich eine ausreichende Erhöhung in der Röntgendichte gewährleistet sein, ohne dass eine übermäßige Dicke der entsprechenden Schichten notwendig ist.

Es ist grundsätzlich möglich, Eigenschaften von metallischen Werkstoffen optimal miteinander zu kombinieren beziehungsweise eine funktionale Schicht mit komplett neuen Werkstoffeigenschaften zu entwickeln. Eine komplexe Struktur des Schichtaufbaus kann insbesondere einen Risswiderstand des Schichtaufbaus im Vergleich zu einfachen Schichten erhöhen und zu einer Unterdrückung und/oder Verzögerung einer Rissinitiierung beitragen. Somit kann der Schichtaufbau ein teilweises pseudo-elastisches Materialverhalten von Stents tolerieren ohne zu versagen, beispielsweise durch Rissentstehung und Rissausbreitung. Stents aus Formgedächtnislegierungen können sich in ihren mechanischen Eigenschaften und ihrem Verformungsverhalten von konventionellen Metallen und Legierungen deutlich unterscheiden. Die vorgeschlagene Erfindung ermöglicht es grundsätzlich, dass die beschichteten Stents aus einer Formgedächtnislegierung bis zu einer Dehnung von 8% elastisch verformt werden können. Außerdem sind die erfindungsgemäß beschriebenen Schichten röntgendicht und erhöhen in Modellversuchen die Röntgensichtbarkeit der beschichteten Substrate im Vergleich zu unbeschichteten Substraten. Zudem weist der Schichtaufbau gute adhäsive Eigenschaften auf. Insbesondere kann eine Delamination der Schichten an der Oberfläche des Stents vermieden oder zumindest weitgehend reduziert werden. Ein weiterer Vorteil der verbesserten Röntgensichtbarkeit besteht in der Möglichkeit, fortlaufende medizinische Kontrollen, insbesondere in zwei bis drei Folgejahren nach der Operation bezüglich einer medizinischen Wirksamkeit, einem Bruch oder einer Beschädigung, zum Beispiel durch Korrosion, durchzuführen.

Durch eine besondere Mikrostruktur der Schichten weisen diese eine hohe Widerstandsfähigkeit gegen Bruch und/oder Rissinitiierung, insbesondere unter mechanischer Last auf. Dies wurde exemplarisch an Untersuchungen mittels dem Zugversuch nachgewiesen: Eine Einlagenschicht aus Tantal zeigte bei einer ausreichenden Röntgensichtbarkeit Risse bei einer Dehnung bis zu 6 %. Dieses Rissnetzwerk vergrößerte sich unter zyklischer Belastung mit 8000 Zyklen. Dem entgegen steht eine Niob-Einlagenschicht, die kein Versagen bei mechanischer Belastung aufwies. Jedoch haben Versuche zur Röntgensichtbarkeit gezeigt, dass das Absorptionsvermögen für Röntgenstrahlung von Niob nicht ausreicht, um die Röntgensichtbarkeit signifikant zu erhöhen. Eine Viellagenschicht aus dem System Ta-Nb löste sowohl das Problem der mechanischen Belastbarkeit als auch die notwendige Röntgensichtbarkeit der Schicht. Des Weiteren kann mit dem Viellagensystem der Spannungszustand der Schicht beeinflusst werden, ohne dabei die Beschichtungsparameter wie z. B. den Gasdruck zu verändern.

Dies ist wichtig, da der Spannungszustand grundsätzlich das Umwandlungsverhalten und - temperaturen beeinflussen kann.

Eine Anzahl der Grenzflächen im Schichtaufbau sowie deren Qualität kann für eine optimale Funktionalität entscheidend sein. Bei einer Betrachtung von vielen Grenzflächen zwischen den unterschiedlichen Schichten des Schichtaufbaus können die Grenzflächen für eine Aufnahme von Energie bei einer Verformung ausgebildet sein. Folglich können die Grenzflächen für eine Unterdrückung einer Rissausbreitung eingerichtet sein. Weist der röntgenopake Schichtaufbau den Gradienten auf, können die Grenzflächen zwischen den Schichten des röntgenopaken Schichtaufbaus sehr kohärent ausgebildet sein, da die Materialien der Schichten jeweils in der gleichen Kristallstruktur nur geringen Änderungen in den Gitterparametern aufwachsen können. Somit ist eine ähnliche Verformung der Schichten zu erwarten, ohne dass zusätzlich neue Risse an den Grenzflächen dieser Lagen auftreten. Dies wird als kohäsives Versagen innerhalb der Schichten bezeichnet.

Im Einzelnen zeigen:

Figuren 1A und 1B     eine schematische Darstellung eines Ausführungsbeispiels eines erfindungsgemäßen Stents in Draufsicht (Figur 1A) und in einer Schnittdarstellung (Figur 1B); und

Figuren 2A bis 2C     schematische Darstellungen von röntgenopaken Schichtaufbauten.

Beschreibung der Ausführungsbeispiele

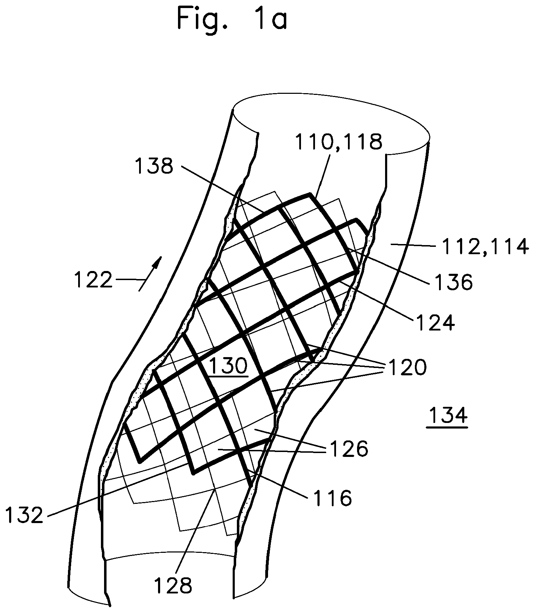

Figur 1A zeigt ein exemplarisches Ausführungsbeispiel eines erfindungsgemäßen Stents in Draufsicht. Der Stent 110 kann eingerichtet sein zur Implantierung in einen Hohlraum 112, insbesondere in ein Gefäß 114, eines menschlichen oder tierischen Körpers.

Der Stent 110 umfasst mindestens einen röhrenförmigen Hohlkörper 116. Der röhrenförmige Hohlkörper 116 kann eine zylindrische Grundform aufweisen. Insbesondere kann es sich bei dem Stent 110 um eine Spiraldrahtprothese 118 handeln. Ein Material des Stents 110 kann in einer Drahtform bereitgestellt sein. Einzelne Drähte 120 des Stents 110 können sich quer, insbesondere senkrecht, zu einer Längserstreckungsachse 122 des Stents 110 erstrecken. Die Drähte 120 können daher eine Mantelfläche 124 des röhrenförmigen Hohlkörpers 116 bilden. Die Drähte 120 können derart angeordnet sein, dass sich zwischen den Drähten 120 Hohlräume 126 ausbilden. Der Stent 110 kann insbesondere eine innere Oberfläche 128 aufweisen, welche einem Innenraum 130 des röhrenförmigen Hohlkörpers 116 zugewandt ist und eine äußere Oberfläche 132 aufweisen, welche einer äußeren Umgebung 134 des röhrenförmigen Hohlkörpers 116 zugewandt ist.

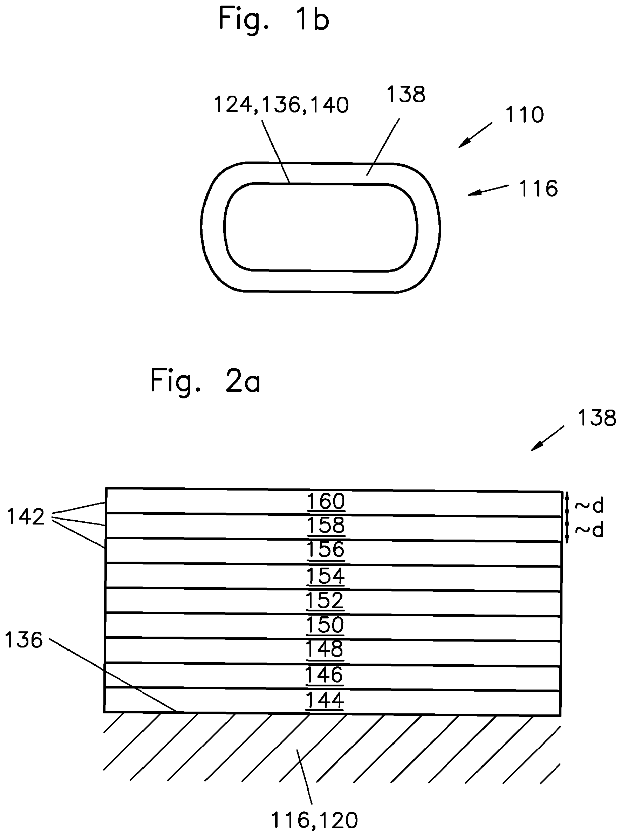

Eine Oberfläche 136 des röhrenförmigen Hohlkörpers 116 ist vollständig mit einer Schicht mit einem röntgenopaken Schichtaufbau 138 beschichtet. Insbesondere kann die Mantelfläche 124 des röhrenförmigen Hohlkörpers 116 beschichtet sein, wie in Figur 1B detailliert dargestellt.

Figur 1B zeigt den Stent 110 gemäß Figur 1A in einer Schnittdarstellung. Insbesondere ist einer der Drähte 120 des Stents 110 in einer Schnittdarstellung gezeigt. Die Oberfläche 136 des röhrenförmigen Hohlkörpers 116, insbesondere des Drahts 120 des röhrenförmigen Hohlkörpers 116 kann vollständig mit dem röntgenopaken Schichtaufbau 138 beschichtet sein. Eine umlaufende Oberfläche 140 der Drähte 120 kann daher mit dem röntgenopaken Schichtaufbau 138 beschichtet sein.

Die Figuren 2A bis 2C zeigen verschiedene Ausführungsbeispiele von röntgenopaken Schichtaufbauten 138. Die röntgenopaken Schichtaufbauten 138 weisen jeweils eine Mehrzahl an übereinanderliegenden Schichten 142 auf. Die Schichten 142 sind jeweils aus mindestens einem Metall hergestellt, welches eine Ordnungszahl von 22 bis 83 aufweist. Zudem weisen die Schichten jeweils eine Schichtdicke d von 1 nm bis 400 nm auf. In ein oder mehreren der Schichten 140 kann das Metall als Reinstoff vorliegen. Alternativ und/oder zusätzlich können ein oder mehrere der Schichten 140 jeweils mindestens eine Legierung aufweisen.

In Figur 2A ist ein röntgenopaker Schichtaufbau 138 dargestellt, welcher einen Gradienten aufweist. Die Schichten 142 des röntgenopaken Schichtaufbaus 138 können sich in Konzentrationen von Komponenten der Legierungen unterscheiden. Eine erste Konzentration einer ersten Komponente kann mit einem zunehmenden Abstand einer der Schichten 140 von der Oberfläche 136 des röhrenförmigen Hohlkörpers 116 abnehmen und eine zweite Konzentration einer zweiten Komponente kann mit einem zunehmenden Abstand der Schichten 140 von der Oberfläche 136 des röhrenförmigen Hohlkörpers 116 zunehmen. Beispielsweise kann eine an die Oberfläche 136 des Stents angrenzende Schicht 144 eine Konzentration $Ta_{0,9}Nb_{0,1}$ aufweisen. Eine an die Schicht 144 angrenzende Schicht 146 kann eine Konzentration $Ta_{0,8}Nb_{0,2}$ aufweisen. Eine an die Schicht 146 angrenzende Schicht 148 kann eine Konzentration $Ta_{0,7}Nb_{0,3}$ aufweisen. Eine an die Schicht 148 angrenzende Schicht 150 kann eine Konzentration $Ta_{0,6}Nb_{0,4}$ aufweisen. Eine an die Schicht 150 angrenzende Schicht 152 kann eine Konzentration $Ta_{0,5}Nb_{0,5}$ aufweisen. Eine an die Schicht 152 angrenzende Schicht 154 kann eine Konzentration $Ta_{0,4}Nb_{0,6}$ aufweisen. Eine an die Schicht 154 angrenzende Schicht 156 kann eine Konzentration $Ta_{0,3}Nb_{0,7}$ aufweisen. Eine an die Schicht 156 angrenzende Schicht 158 kann eine Konzentration $Ta_{0,2}Nb_{0,8}$ aufweisen. Eine an die Schicht 158 angrenzende Schicht 160 kann eine Konzentration $Ta_{0,1}Nb_{0,9}$ aufweisen.

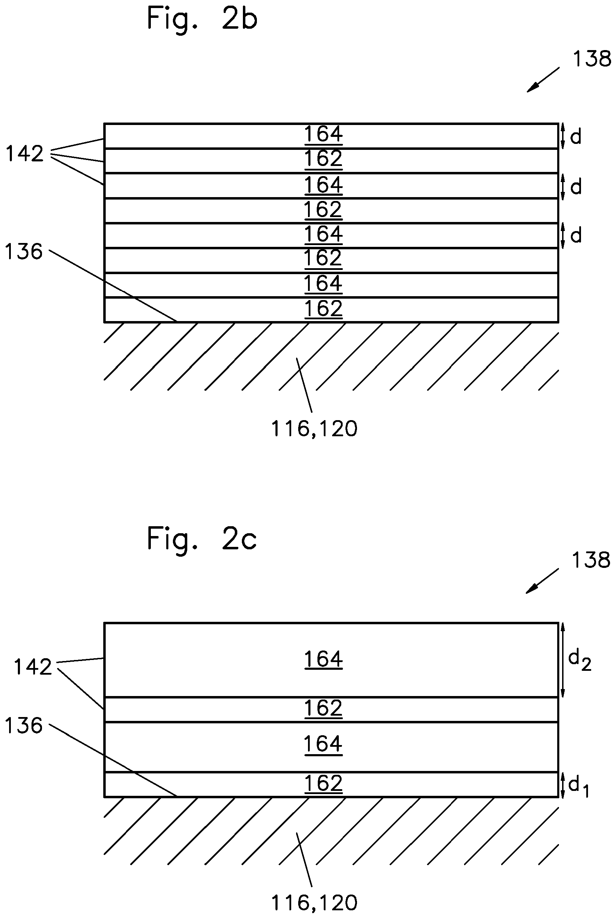

In Figur 2B ist ein weiterer röntgenopaker Schichtaufbau 138 dargestellt. Der röntgenopake Schichtaufbau 138 kann mindestens zwei erste Schichten 162 und mindestens eine zweite Schicht 164 aufweisen. Die zweite Schicht 164 kann zwischen den ersten Schichten 162 angeordnet sein. Insbesondere kann der röntgenopake Schichtaufbau 138 mehrere der ersten Schichten 162 aufweisen und mehrere der zweiten Schichten 164, welche jeweils abwechselnd übereinander angeordnet sind. Die erste Schicht 162 kann beispielsweise Tantal aufweisen und die zweite Schicht kann beispielsweise Titan oder Niob aufweisen.

In Figur 2C ist ein weiterer röntgenopaker Schichtaufbau 138 dargestellt. Der röntgenopake Schichtaufbau 138 gemäß Figur 2C unterscheidet sich von dem röntgenopaken Schichtaufbau 138 gemäß Figur 2B in der Schichtdicke der ersten Schichten 162 und der zweiten Schichten 164. Die ersten Schichten 162 können jeweils eine Schichtdicke $d_1$ aufweisen und die zweiten Schichten 164 können jeweils eine Schichtdicke $d_2$ aufweisen. Ein Verhältnis $d_1:d_2$ kann mittels eines Beschichtungsprozesses einstellbar sein.

Bezugszeichenliste

| | |
|---|---|
| 110 | Stent |
| 112 | Hohlraum |
| 114 | Gefäß |
| 116 | röhrenförmiger Hohlkörper |
| 118 | Spiraldrahtprothese |
| 120 | Draht |
| 122 | Längserstreckungsachse |
| 124 | Mantelfläche |
| 126 | Hohlraum |
| 128 | innere Oberfläche |
| 130 | Innenraum |
| 132 | äußere Oberfläche |

| 134 | äußere Umgebung |
| 136 | Oberfläche |
| 138 | röntgenopaker Schichtaufbau |
| 140 | umlaufende Oberfläche |
| 142 | Schicht |
| 144 | Schicht |
| 146 | Schicht |
| 148 | Schicht |
| 150 | Schicht |
| 152 | Schicht |
| 154 | Schicht |
| 156 | Schicht |
| 158 | Schicht |
| 160 | Schicht |
| 162 | erste Schicht |
| 164 | zweite Schicht |

## Patentansprüche

1. Stent (110) zur Implantierung in einen Hohlraum (112) eines menschlichen oder tierischen Körpers, wobei der Stent (110) mindestens einen röhrenförmigen Hohlkörper (116) umfasst, wobei der röhrenförmige Hohlkörper (116) mindestens eine Oberfläche (136) aufweist, wobei die Oberfläche (136) vollständig mit einem röntgenopaken Schichtaufbau (138) beschichtet ist, welcher eine Mehrzahl an jeweils übereinanderliegenden Schichten (142) aufweist, wobei der röntgenopake Schichtaufbau (138) mindestens zehn Schichten aufweist, wobei die Schichten (142) jeweils aus mindestens einem Metall hergestellt sind, welches eine Ordnungszahl von 22 bis 83 aufweist, wobei das Metall ausgewählt ist aus der Gruppe bestehend aus: Wolfram, Niob, Tantal, Titan, wobei die Schichten (142) jeweils eine Schichtdicke d im Bereich von 1 nm bis 400 nm aufweisen.

2. Stent (110) nach dem vorhergehenden Anspruch, wobei in einer oder mehreren der Schichten (142) das Metall als Reinstoff vorliegt.

3. Stent (110) nach einem der vorhergehenden Ansprüche, wobei eine oder mehrere der Schichten (142) jeweils mindestens eine Legierung aufweisen, wobei die Legierung ausgewählt ist aus der Gruppe bestehend aus: einer einphasigen binären $Ta_{1-x}Nb_x$-Legierung mit $0<x<1$, einer einphasigen binären $Ti_{1-x}Ta_x$-Legierung mit $0<x<1$, einer ternären $Ta_{1-x-y}Ti_xNb_y$- Legierung mit $0<x<1$ und mit $0<y<1$.

4. Stent (110) nach dem vorhergehenden Anspruch, wobei der röntgenopake Schichtaufbau (138) einen Gradienten aufweist, wobei sich die Schichten (142) des röntgenopaken Schichtaufbaus (138) in Konzentrationen der Komponenten der Legierungen unterscheiden, wobei eine erste Konzentration einer ersten Komponente mit einem zunehmenden Abstand einer der Schichten (142) von der Oberfläche (136) des röhrenförmigen Hohlkörpers (116) abnimmt und wobei eine zweite Konzentration einer zweiten Komponente mit dem zunehmenden Abstand einer der Schichten (142) von der Oberfläche (136) des röhrenförmigen Hohlkörpers (116) zunimmt.

5. Stent (110) nach einem der vorhergehenden Ansprüche, wobei der röntgenopake Schichtaufbau (138) eine Schichtaufbaudicke von 500 nm bis 50 $\mu$m aufweist.

6. Stent (110) nach einem der vorhergehenden Ansprüche, wobei der röntgenopake Schichtaufbau (138) mindestens zwei erste Schichten (162) mit einem ersten Material und mindestens eine zweite Schicht (164) mit einem zweiten Material aufweist, wobei die zweite Schicht (164) zwischen den ersten Schichten (162) angeordnet ist.

7. Stent (110) nach dem vorhergehenden Anspruch, wobei die ersten Schichten (162) Tantal aufweisen und wobei die zweite Schicht (164) Titan oder Niob aufweist.

8. Stent (110) nach einem der vorhergehenden Ansprüche, wobei die ersten Schichten (162) eine erste Schichtdicke $d_1$ aufweisen, wobei die zweite Schicht (164) eine zweite Schichtdicke $d_2$ aufweist, wobei ein Verhältnis $d_1:d_2$ mittels eines Beschichtungsprozesses einstellbar ist.

9. Verfahren zur Herstellung eines röntgenopaken Schichtaufbaus (138) auf einem Stent (110) zur Implantierung in einen Hohlraum (112) eines menschlichen oder tierischen Körpers, wobei das Verfahren die folgenden Schritte umfasst:

    a) Bereitstellen eines röhrenförmigen Hohlkörpers (116), wobei der röhrenförmige Hohlkörper (116) mindestens eine Oberfläche (136) aufweist; und
    b) Aufbringen eines röntgenopaken Schichtaufbaus (138) auf die Oberfläche (136), derart, dass die Oberfläche vollständig bedeckt wird, wobei der röntgenopake Schichtaufbau (138) eine Mehrzahl an jeweils übereinanderliegenden Schichten (142) aufweist, wobei der röntgenopake Schichtaufbau (138) mindestens zehn Schichten aufweist, wobei die Schichten (142) jeweils aus mindestens einem Metall hergestellt sind, welches eine Ordnungszahl von 22 bis 83 aufweist, wobei das Metall ausgewählt ist aus der Gruppe bestehend aus: Wolfram, Niob, Tantal, Titan, wobei die Schichten (142) jeweils eine Schichtdicke d im Bereich von 1 nm bis 400 nm aufweisen.

10. Verfahren nach dem vorhergehenden Anspruch, wobei die Schichten (142) jeweils nacheinander mittels physikalischer Gasphasenabscheidung hergestellt werden.

11. Verfahren nach dem vorhergehenden Anspruch, wobei die physikalische Gasphasenabscheidung unter Anwendung einer Substratvorspannung erfolgt.

**Claims**

1. Stent (110) for implanting into a cavity (112) of a human or animal body, wherein the stent (110) comprises at least one tubular hollow body (116), wherein the tubular hollow body (116) has at least one surface (136), wherein the surface (136) is coated completely with an X-ray-opaque layer structure (138) which comprises a plurality of superposed layers (142), wherein the X-ray-opaque layer structure (138) has at least ten layers, wherein the layers (142) are each made of at least one metal which has an atomic number of from 22 to 83, wherein the metal is selected from the group consisting of: tungsten, niobium, tantalum, titanium, wherein the layers (142) each have a layer thickness d in the range from 1 nm to 400 nm.

2. Stent (110) according to the preceding claim, wherein the metal is present as pure material in one or more of the layers (142).

3. Stent (110) according to either of the preceding claims, wherein one or more of the layers (142) each comprise at least one alloy, wherein the alloy is selected from the group consisting of: a single-phase binary $Ta_{1-x}Nb_x$ alloy where $0<x<1$, a single-phase binary $Ti_{1-x}Ta_x$ alloy where $0<x<1$, a ternary $Ta_{1-x-y}Ti_xNb_y$ alloy where $0<x<1$ and $0<y<1$.

4. Stent (110) according to the preceding claim, wherein the X-ray-opaque layer structure (138) has a gradient, wherein the layers (142) of the X-ray-opaque layer structure (138) differ in respect of the concentrations of the components of the alloys, wherein a first concentration of a first component decreases with increasing distance of one of the layers (142) from the surface (136) of the tubular hollow body (116) and wherein a second concentration of a second component increases with increasing distance of one of the layers (142) from the surface (136) of the tubular hollow body (116).

5. Stent (110) according to any of the preceding claims, wherein the X-ray-opaque layer structure (138) has a layer structure thickness of from 500 nm to 50 $\mu$m.

6. Stent (110) according to any of the preceding claims, wherein the X-ray-opaque layer structure (138) comprises at least two first layers (162) comprising a first material and at least one second layer (164) comprising a second material, wherein the second layer (164) is arranged between the first layers (162).

7. Stent (110) according to the preceding claim, wherein the first layers (162) comprise tantalum and wherein the second layer (164) comprises titanium or niobium.

8. Stent (110) according to any of the preceding claims, wherein the first layers (162) have a first layer thickness $d_1$, wherein the second layer (164) has a second layer thickness $d_2$, wherein a ratio $d_1:d_2$ can be set by means of a coating process.

9. Process for producing an X-ray-opaque layer structure (138) on a stent (110) for implanting into a cavity (112) of a human or animal body, wherein the process comprises the following steps:

   a) provision of a tubular hollow body (116), wherein the tubular hollow body (116) has at least one surface (136); and
   b) application of an X-ray-opaque layer structure (138) to the surface (136) in such a way that the surface is completely covered, wherein the X-ray-opaque layer structure (138) comprises a plurality of superposed layers (142), wherein the X-ray-opaque layer structure (138) has at least ten layers, wherein the layers (142) are each made of at least one metal which has an atomic number of from 22 to 83, wherein the metal is selected from the group consisting of: tungsten, niobium, tantalum, titanium, wherein the layers (142) each have a layer thickness d in the range from 1 nm to 400 nm.

10. Process according to the preceding claim, wherein the layers (142) are produced in succession by means of physical vapour deposition.

11. Process according to the preceding claim, wherein the physical vapour deposition is carried out with prestressing of the substrate.

## Revendications

1. Stent (110) destiné à être implanté dans une cavité (112) d'un corps humain ou animal, le stent (110) comprenant au moins un corps creux tubulaire (116), le corps creux tubulaire (116) comportant au moins une surface (136), la surface (136) étant entièrement revêtue d'une structure de couches (138), opaque aux rayons X, qui comporte une pluralité de couches (142) superposées, la structure de couches (138), opaque aux rayons X, comportant au moins dix couches, les couches (142) étant chacune au moins en un métal qui a un numéro atomique allant de 22 à 83, le métal étant choisi dans le groupe comprenant : le tungstène, le niobium, le tantale, le titane, les couches (142) ayant chacune une épaisseur d dans la plage de 1 nm à 400 nm.

2. Stent (110) selon la revendication précédente, le métal étant présent dans une ou plusieurs des couches (142) sous la forme d'une substance pure.

3. Stent (110) selon l'une des revendications précédentes, une ou plusieurs des couches (142) comportant chacune au moins un alliage, l'alliage étant choisi dans le groupe comprenant : un alliage binaire monophasé $Ta_{1-x}Nb_x$ avec $0 < x < 1$, un alliage binaire monophasé $Ti_{1-x}Ta_x$ avec $0 < x < 1$, un alliage ternaire $Ta_{1-x-y}Ti_xNb_y$ avec $0 < x < 1$, et $0 < y < 1$.

4. Stent (110) selon la revendication précédente, la structure de couches (138), opaque aux rayons X, présentant un gradient, les couches (142) de la structure de couche (138), opaque aux rayons X, différant par les concentrations des composants des alliages, une première concentration d'un premier composant diminuant à mesure qu'une distance de l'une des couches (142) à la surface (136) du corps creux tubulaire (116) augmente et une deuxième concentration d'un deuxième composant augmentant à mesure que la distance de l'une des couches (142) à la surface (136) du corps tubulaire creux (116) augmente.

5. Stent (110) selon l'une des revendications précédentes, la structure de couches (138), opaque aux rayons X, ayant une épaisseur de 500 nm à 50 $\mu$m.

6. Stent (110) selon l'une des revendications précédentes, la structure de couches (138), opaque aux rayons X, comportant au moins deux premières couches (162) comprenant un premier matériau et au moins une deuxième couche (164) comprenant un deuxième matériau, la deuxième couche (164) étant disposée entre les premières couches (162).

7. Stent (110) selon la revendication précédente, les premières couches (162) comprenant du tantale et la deuxième couche (164) comprenant du titane ou du niobium.

8. Stent (110) selon l'une des revendications précédentes, les premières couches (162) ayant une première épaisseur $d_1$, la deuxième couche (164) ayant une deuxième épaisseur $d_2$, un rapport $d_1:d_2$ pouvant être fixé au moyen d'un procédé de revêtement.

**9.** Procédé de production d'une structure de couches (138), opaque aux rayons X, sur un stent (110) destiné à être implanté dans une cavité (112) d'un corps humain ou animal, le procédé comprenant les étapes suivantes :

a) fournir un corps creux tubulaire (116), le corps creux tubulaire (116) comportant au moins une surface (136) ; et
b) appliquer une structure de couches (138), opaque aux rayons X, sur la surface (136) de telle sorte que la surface soit entièrement recouverte, la structure de couches (138), opaque aux rayons X, comportant une pluralité de couches (142) superposées, la structure de couches (138), opaque aux rayons X, comportant au moins dix couches, les couches (142) étant chacune en au moins un métal qui a un numéro atomique de 22 à 83, le métal étant choisi dans le groupe comprenant : le tungstène, le niobium, le tantale, le titane, les couches (142) ayant chacune une épaisseur d dans la plage de 1 nm à 400 nm.

**10.** Procédé selon la revendication précédente, les couches (142) étant chacune produites l'une après l'autre par dépôt physique en phase gazeuse.

**11.** Procédé selon la revendication précédente, le dépôt physique en phase gazeuse étant effectué à l'aide d'une précontrainte du substrat.

# Fig. 1a

# Fig. 1b

124,136,140    138    110
116

# Fig. 2a

138

142

| | |
|---|---|
| 160 | ~d |
| 158 | ~d |
| 156 | |
| 154 | |
| 152 | |
| 150 | |
| 148 | |
| 146 | |
| 144 | |

136

116,120

## Fig. 2b

138

142

164    d

162

164    d

162

164    d

136

162

164

162

116,120

## Fig. 2c

138

142

164    d₂

162

136

164

162    d₁

116,120

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0709068 A2
- DE 69913342 T2
- DE 102007030751 B4
- EP 0824900 A2
- KR 101791337B1
- US 6638301 B1
- US 2002138130 A1
- WO 2015168016 A1

**In der Beschreibung aufgeführte Nicht-Patentliteratur**

- **C. BECHTOLD ; R. LIMA DE MIRANDA ; C. CHLUBA ; C. ZAMPONI ; E. QUANDT.** Method for Fabricating Miniaturized NiTi Self-Expandable Thin Film Devices with Increased Radiopacity. *Shap. Mem. Superelasticity,* 2016, vol. 2, 391-398
- **A. C. KNEISSL ; K. MEHRABI ; M. BRUNCKO ; B. J. MCKAY ; D. UHLENHAUT.** Characterization and properties of NiTi(W) and CuAlNi shape memory alloys. *International Journal of Materials Research,* 2009, vol. 100 (8), 1038-1045
- **IN C. PARK ; S. KIM, H.-E ; KIM, T.-S. JANG.** Mechanically stable tantalum coating on a nano-roughened NiTi stent for enhanced radiopacity and biocompatibility. *Surface and Coatings Technology,* 2016, vol. 305, 139-145
- **C. HEßING ; J. FRENZEL ; M. POHL ; S. SHABALOVSKAYA.** Effect of martensitic transformation on the performance of coated NiTi surfaces. *Materials Science and Engineering: A,* 2008, vol. 486, 461-469